# EUROPÄISCHE PATENTANMELDUNG

(11) **EP 2 251 713 A1**
(43) Veröffentlichungstag der Anmeldung: **17.11.2010**
(21) Anmeldenummer: 09159988.6
(22) Anmeldetag: 12.05.2009
(51) Int. Cl.: G01T 1/20, H01L 27/30

(54) **Detektor zum Nachweis ionisierender Strahlung**

(71) Anmelder: Ruprecht-Karls-Universität Heidelberg, 69117 Heidelberg (DE)
(72) Erfinder: Hesser, Jürgen, 69118 Heidelberg (DE); Wenz, Frederik, 69120 Heidelberg (DE); Gretz, Norbert, 68259 Mannheim (DE)
(74) Vertreter: Hörschler, Wolfram Johannes

(57) **Zusammenfassung**

Es wird ein Detektor (110) zum Nachweis ionisierender Strahlung (116) vorgeschlagen. Der Detektor (110) umfasst mindestens einen Szintillator (112), welcher eingerichtet ist, um die ionisierende Strahlung (116) in elektromagnetische Strahlung (118) umzuwandeln, insbesondere in sichtbares, ultraviolettes oder infrarotes Licht. Der Detektor (110) umfasst weiterhin mindestens ein organisches fotovoltaisches Element (114), welches eingerichtet ist, um die elektromagnetische Strahlung (118) in mindestens ein elektrisches Signal (120) umzuwandeln.

## Beschreibung

### Gebiet der Erfindung

Die Erfindung betrifft einen Detektor zum Nachweis ionisierender Strahlung sowie einen derartigen Detektor umfassende Beschichtungen, Bestrahlungssysteme und Reinigungsvorrichtungen. Weiterhin betrifft die Erfindung ein Verfahren zur Detektion ionisierender Strahlen sowie ein Verfahren zur Überwachung eines Bestrahlungssystems, jeweils unter Verwendung eines erfindungsgemäßen Detektors. Derartige Vorrichtungen und Verfahren werden allgemein beispielsweise im Bereich der Strahlenhygiene eingesetzt, beispielsweise in der medizinischen Therapeutik, der medizinischen Diagnostik, der Nukleartechnologie oder der Forschung.

### Stand der Technik

In vielen Bereichen der Medizin, der Naturwissenschaften und der Technik werden Substanzen oder Vorrichtungen eingesetzt, welche ionisierende Strahlung erzeugen. Derartige Substanzen werden allgemein auch als "radioaktive Substanzen" bezeichnet. Je nach Art der ionisierenden Strahlung, bei welcher es sich beispielsweise um Partikelstrahlung und/oder um elektromagnetische Strahlung handeln kann, werden α-, β- und γ-Strahler unterschieden. Im weitesten Sinne können jedoch auch Neutronenquellen unter "radioaktive Substanzen" subsumiert werden, da Neutronen zwar selbst in der Regel keine ionisierende Wirkung aufweisen, jedoch eine aktivierende Wirkung und somit ebenfalls zu einer Ionisierung von bestrahlten Materialien führen können. Unter ionisierender Strahlung wird somit im Rahmen der vorliegenden Erfindung allgemein eine Strahlung verstanden, welche direkt oder indirekt ionisierende Wirkung auf Materialen ausüben kann, beispielsweise auf Körpergewebe, und welche eine oder mehrere der Strahlen, ausgewählt aus α-, β-, γ-, Röntgen- und Neutronen-Strahlung umfasst.

Ionisierende Strahlung der beschriebenen Art wird beispielsweise in der Strahlentherapeutik oder der Strahlendiagnostik in der Medizin eingesetzt. Diese Zweige der Medizin gewinnen insbesondere im Rahmen der Onkologie zunehmen an Bedeutung, jedoch auch in anderen Bereichen der Medizin. Weiterhin wird ionisierende Strahlung im Bereich der Materialwissenschaften und Technik eingesetzt, beispielsweise in Form von Röntgen- oder γ-Strahlung zur Materialprüfung, beispielsweise im Brückenbau oder in der Fahrzeugtechnik. Weitere Einsatzgebiete ionisierender Strahlung liegen in der Nukleartechnik, beispielsweise im Bereich von Kraftwerken, oder in der Biologie, beispielsweise bei der Verwendung als Markermaterialien.

Beim Umgang mit ionisierender Strahlung oder mit Substanzen oder Vorrichtungen, welche ionisierende Strahlung erzeugen, ist grundsätzlich äußerste Vorsicht geboten, da in der Regel weder die ionisierende Strahlung selbst noch die diese ionisierende Strahlung erzeugenden Substanzen mit menschlichen Sinnen wahrnehmbar sind. Somit ist zum einen eine Strahlenbelastung der beteiligten Personen bzw. Lebewesen zu überwachen. Andererseits ist jedoch auch die Funktionsfähigkeit der Strahlenquellen zumindest in regelmäßigen Abständen zu kontrollieren. Weiterhin muss sichergestellt werden, dass Gegenstände oder Lebewesen nicht ungewollt und insbesondere unbemerkt mit ionisierende Strahlung erzeugenden Substanzen kontaminiert werden. Dieser Aufgabenbereich wird insbesondere auch als "Strahlenhygiene" bezeichnet.

Aus dem Stand der Technik sind zahlreiche aufwendige Detektorsystem bekannt, welche für einen oder mehrere der genannten Zwecke eingesetzt werden können. So werden beispielsweise im Strahlenschutz zur Überwachung einer Strahlendosis in vielen Fällen Dosimeter eingesetzt, welche nach verschiedenen Wirkprinzipien arbeiten können und welche in der Regel vergleichsweise aufwändig hergestellt, zu bedienen und auszuwerten sind. Zur Überwachung von Strahlenkontaminationen werden insbesondere Detektoren, wie beispielsweise Geiger-Müller-Zählrohre, eingesetzt. Weiterhin werden Filme eingesetzt, welche zur Überwachung einer Dosis und/oder einer räumlichen Verteilung ionisierender Strahlung genutzt werden können. Bekannte Detektoren sind jedoch allgemein in der Regel teuer, technisch aufwändig und können in vielen Fällen erst dann eingesetzt werden, wenn ein konkreter Verdacht auf eine Kontamination mit ionisierende Strahlung erzeugenden Substanzen besteht.

### Aufgabe der Erfindung

Es ist daher eine Aufgabe der vorliegenden Erfindung, einen Detektor bereitzustellen, welcher die Nachteile bekannter Detektoren vermeidet. Insbesondere soll der Detektor einfach und kostengünstig herstellbar sein, flexibel und vielseitig verwendbar sein und insbesondere im Bereich der Strahlenhygiene eingesetzt werden können.

### Offenbarung der Erfindung

Diese Aufgabe wird mit den Vorrichtungen und Verfahren gemäß den unabhängigen Ansprüchen gelöst. Vorteilhafte Weiterbildungen der Erfindung, welche einzeln oder in Kombination realisiert werden können, sind in den abhängigen Ansprüchen dargestellt.

Ein grundlegender Gedanke der vorliegenden Erfindung besteht darin, zum Nachweis der ionisierenden Strahlung Szintillatoren in Kombination mit organischen fotovoltaischen Elementen zu verwenden. Organische fotovoltaische Elemente lassen sich, im Gegensatz zu herkömmlichen Lichtdetektoren, die in Kombination mit üblichen Szintillatoren eingesetzt werden, in nahezu beliebiger Form erzeugen, lassen sich flexibel ausgestalten, sind großflächig herstellbar und damit insgesamt zur Lösung der oben genannten Aufgabe gut geeignet.

Es wird daher ein Detektor zum Nachweis ionisierender Strahlung im Sinne der obigen Definition vorgeschlagen, welcher mindestens einen Szintillator und mindestens ein organisches fotovoltaisches Element umfasst. Der Szintillator ist eingerichtet, um ionisierende Strahlung in elektromagnetische Strahlung umzuwandeln, insbesondere in sichtbares und/oder ultraviolettes und/oder infrarotes Licht. Diese elektromagnetische Strahlung soll derart ausgestaltet und/oder von dem Szintillator ausgegeben werden, dass diese zumindest teilweise von dem organischen fotovoltaischen Element aufgenommen bzw. detektiert werden kann. Das organische fotovoltaische Element ist eingerichtet, um die elektromagnetische Strahlung in mindestens ein elektrisches Signal umzuwandeln. Dieses elektrische Signal kann von dem Detektor ausgegeben werden, beispielsweise über eine Schnittstelle, oder kann, alternativ oder zusätzlich, auch als internes Signal verwendet werden und beispielsweise an weitere Elemente des Detektors weitergegeben werden, beispielsweise an das optionale und unten noch näher erläuterte Anzeigenelement.

Der Detektor kann insbesondere eingerichtet sein, um direkt oder indirekt, beispielsweise über ein Anzeigenelement, mindestens ein Ausgangssignal entsprechend des elektrischen Signals zu erzeugen. So kann dieses Ausgangssignal beispielsweise ein elektrisches Ausgangssignal umfassen, welches beispielsweise über eine Schnittstelle an einen Benutzer und/oder eine weitere Vorrichtung ausgegeben werden kann. Alternativ oder zusätzlich kann das Ausgangssignal, wie unten näher ausgeführt wird, auch mindestens ein optisches und/oder akustisches und/oder haptisches und/oder auf andere Weise für einen Benutzer wahrnehmbares Signal umfassen, beispielsweise ein von einem Anzeigenelement ausgegebenes Signal.

Unter einem Szintillator ist dabei im Rahmen der vorliegenden Erfindung allgemein ein Material und/oder ein Element zu verstehen, welches beim Durchgang und/oder Auftreffen der ionisierenden Strahlung, bei welcher es sich beispielsweise um Partikelstrahlung und/oder ionisierende elektromagnetische Strahlung handeln kann, angeregt wird und diese Anregungsenergie in Form der elektromagnetischen Strahlung, beispielsweise im ultravioletten und/oder sichtbaren und/oder infraroten Spektralbereich, wieder abgibt. Beispielsweise kann dies mit dem Vorgang einer Konversion verbunden sein, wenn die ionisierende Strahlung ebenfalls elektromagnetische Strahlung umfasst. In diesem Fall kann beispielsweise eine Konversion der elektromagnetischen Strahlung hin zu längerwelliger elektromagnetischer Strahlung erfolgen. Im Fall von Partikelstrahlung als ionisierender Strahlung erfolgt eine Umwandlung in elektromagnetische Strahlung.

Der Szintillator kann grundsätzlich anorganische und/oder organische Szintillatormaterialien umfassen. Besonders bevorzugt ist es, wenn der Szintillator mindestens ein organisches Szintillatormaterial umfasst oder vollständig aus einem organischen Szintillatormaterial besteht, da organische Szintillatormaterialien grundsätzlich vergleichsweise einfach herzustellen sind und da bei der Verwendung organischer Szintillatormaterialien beispielsweise, wie unten näher erläutert wird, rein organische oder nahezu ausschließlich organische Schichtaufbauten erzeugt werden können. Insbesondere kann bei der Verwendung mindestens eines organischen Szintillatormaterials eine Prozesskompatibilität mit der Herstellung des organischen fotovoltaischen Elements bestehen, was allgemein die Herstellung vereinfacht. Alternativ oder zusätzlich können jedoch grundsätzlich auch anorganische Szintillatoren verwendet werden. Ein organisches Szintillatormaterial kann beispielsweise einem organischen Farbstoff und/oder ein Polymer oder ähnlichen Materialien umfassen, welche eingerichtet sind, um bei einer entsprechenden Anregung durch die ionisierende Strahlung die elektromagnetische Strahlung zu emittieren. Auch dotierte organische Materialien können eingesetzt werden, beispielsweise organische Matrixmaterialien, welche mit mindestens einem organischen und/oder anorganischen Dotierstoff dotiert sind. Auch Mischungen organischer und anorganischer Szintillatormaterialien sind möglich. Beispiele organischer Szintillatormaterialien sind Anthrazen, Stilben, Terpenyl, Diphenylazethylen, Polyvinlytoluol, Toluol oder Xylol.

Anorganische Szintillatoren können beispielsweise Kristalle umfassen, welche mit Aktivatorzentren und/oder Farbzentren dotiert sein können. Ionisierende Strahlung kann in diesem Festkörper freie Elektronen, freie Löcher oder Elektron-Loch-Paare, insbesondere Exzitonen erzeugen. Derartige Anregungszustände können sich in dem Szintillatormaterial fortbewegen, bis die Anregungszustände, in der Regel an Aktivatorzentren, zerfallen und dabei die elektromagnetische Strahlung aussenden. Beispiele derartiger anorganischer Szintillatormaterialien sind Zinksulfid, Natriumiodid, Bismutgermanat, Lutetiumoxyorthosilicat oder Kombinationen der genannten und/oder anderer Szintillatormaterialien.

Organische Szintillatoren können grundsätzlich Kristalle und/oder Flüssigkeiten und/oder polymere Festkörper umfassen. Der Mechanismus der Szintillation in organischen Szintillatoren kann beispielsweise auf einer Anregung von Molekülzuständen beruhen, beispielsweise in Form von Fluoreszenzstoffen, wobei die angeregten Zustände beim Zerfall beispielsweise ultraviolette Strahlung imitieren können. Weiterhin können, wie auch in den anorganischen Szintillatormaterialien, auch weitere Konversionsmaterialien enthalten sein, beispielsweise so genannte "Wellenlängeschieber", welche beispielsweise kürzerwellige elektromagnetische Strahlung in längerwellige elektromagnetische Strahlung umwandeln können.

Grundsätzlich kann das Szintillatormaterial vorzugsweise in fester Form vorliegen, vorzugsweise in flexibler Form, beispielsweise als eine oder mehrere Szintillatorfolien. Grundsätzlich sind jedoch auch andere Aggregatszustände alternativ oder zusätzlich einsetzbar, wie beispielsweise Flüssigszintillatoren.

Unter einem organischen fotovoltaischen Element wird allgemein im Rahmen der vorliegenden Erfindung ein Element verstanden, welches eingerichtet ist, um elektromagnetische Strahlungen in elektrische Signale umzuwandeln und welches mindestens ein organisches Material umfasst. Bei dem organischen Material kann es sich insbesondere um mindestens eine organische Schicht handeln, wobei Mehrschicht-Aufbauten bevorzugt sind. Beispielsweise können mehrere organische Funktionsschichten umfasst sein, beispielsweise ein oder mehrere Konversionsschichten zur Umwandlung der elektromagnetischen Strahlung in angeregte Zustände in dem organischen Material und/oder ein oder mehrere Ladungstransportschichten zum Transport von positiven oder negativen Ladungen. Weiterhin kann das organische fotovoltaische Element eine, vorzugsweise zwei oder mehrere Elektroden umfassen, beispielsweise mindestens eine Kathode und mindestens eine Anode, von denen vorzugsweise mindestens eine zumindest teilweise transparent für elektromagnetische Strahlung ausgestaltet ist, insbesondere in dem von dem Szintillator emittierten Wellenlängenbereich. Anstelle eines rein organischen Aufbaus des organischen fotovoltaischen Elements, beispielsweise mit Ausnahme einer oder mehrerer der Elektroden (deren Materialen grundsätzlich auch aus anorganischen Materialen bestehen können), können auch Hybrid-Elemente eingesetzt werden, beispielsweise Elemente, welche anorganische und organische Materialen kombinieren. So können beispielsweise Schichtaufbauten mit einer oder mehreren anorganischen Schichten mit einer oder mehreren organischen Schichten kombiniert werden.

Besonders bevorzugt ist es, wenn das organische fotovoltaische Element mindestens eine organische Solarzelle und/oder mindestens eine organische Fotodiode umfasst. Insbesondere können mindestens ein organisches Solarzellen-Array und/oder mindestens ein organisches Fotodioden-Array vorgesehen sein. Unter einem Array ist dabei allgemein eine ein-, zwei- oder drei-dimensionale Anordnung mehrerer aktiver Felder zu verstehen, beispielsweise mehrerer organischer Fotodioden-Felder und/oder mehrerer organischer Solarzellen-Felder. Unter einer organischen Solarzelle kann allgemein im Rahmen der vorliegenden Erfindung eine Solarzelle verstanden werden, welche mindestens einen Werkstoff der organischen Chemie aufweist. Bei dem organischen Werkstoff kann es sich beispielsweise um einen niedermolekularen organischen Werkstoff und/oder einen PolymerWerkstoff handeln, wobei auch Kombinationen der genannten Werkstoffe möglich sind und/oder Kombinationen mit anorganischen Werkstoffen. Insbesondere können organische Werkstoffe mit konjugierten π-Elektronensystemen verwendet werden, insbesondere konjugierte Polymere. Dabei können auch Akzeptor-Materialien eingesetzt werden, beispielsweise Fullerene. Insbesondere können Donator-Materialien und Akzeptor-Materialien miteinander zu einem organischen Solarzellen-Aufbau kombiniert werden. Die organischen Solarzellen können beispielsweise in nass-chemischen Prozessen und/oder in Vakuum-Prozessen hergestellt werden. Bezüglich möglicher Ausgestaltungen der organischen Solarzellen kann grundsätzlich auf einschlägige Literatur verwiesen werden, da bekannte und teilweise kommerziell erhältliche organische Solarzellen grundsätzlich auch im Rahmen der vorliegenden Erfindung einsetzbar sind.

Der Detektor kann weiterhin mindestens eine elektronische Schaltung umfassen, beispielsweise zur Verstärkung und/oder zumindest teilweisen Verarbeitung und/oder zur Ausgabe des mindestens einen elektrischen Signals. So kann der Detektor beispielsweise mindestens einen Verstärker zur Verstärkung des elektrischen Signals umfassen. Auch dieser Verstärken und/oder andere elektrischen Komponenten des Detektors können wiederum ganz oder teilweise unter Verwendung organischer Werkstoffe hergestellt sein. So kann beispielsweise der Verstärker insbesondere mindestens ein organisches elektronisches Element umfassen, vorzugsweise mindestens einen organischen Transistor. Auch auf diese Weise kann der oben beschriebene, bevorzugt im Wesentlichen gesamt-organische Aufbau gefördert werden, beispielsweise indem der Szintillator zumindest teilweise als organischer Szintillator aufgebaut ist, indem das organische fotovoltaische Element verwendet wird und indem auch eine Elektronik zumindest teilweise als organische Elektronik ausgestaltet ist. Weiterhin kann, wie unten näher ausgeführt wird, mindestens ein organisches Anzeigenelement vorgesehen sein. Wie oben dargestellt, begünstigt der genannte, im Wesentlichen gesamt-organische Aufbau insgesamt die Herstellung des Detektors, da für die einzelnen Elemente des Detektors ähnliche Produktionstechniken eingesetzt werden können. Organische Elektronik-Bauelmente, insbesondere organische Transistoren, sind allgemein Elemente, welche für den Stromtransport auf ein oder mehrere organische Materialien zurückgreifen, beispielsweise ein oder mehrere organische Leitermaterialien und/oder Halbleitermaterialien. Auch derartige organische elektronische Elemente sind grundsätzlich aus dem Stand der Technik bekannt.

Der Detektor kann insbesondere eingerichtet sein, um aus dem elektrischen Signal eine Dosisinformation über eine Strahlungsdosis der ionisierenden Strahlung zu erzeugen. So kann der Detektor beispielsweise das mindestens eine elektrische Signal direkt oder indirekt, vollständig oder teilweise in die genannte Dosisinformation umwandeln. Dies kann beispielsweise über eine entsprechende Auswertelektronik und/oder eine Datenverarbeitungsvorrichtung erfolgen, welche auch ganz oder teilweise Bestandteil des Detektors sein kann, welche jedoch auch ganz oder teilweise außerhalb des Detektors angeordnet sein kann. Beispielsweise kann das elektrische Signal direkt oder indirekt in die Dosisinformation umgewandelt werden, beispielsweise mittels einer oder mehrerer entsprechender Kalibrationsinformationen. Diese Kalibrationsinformationen können beispielsweise in einem Datenspeicher hinterlegt sein, welcher ebenfalls ganz oder teilweise in dem Detektor enthalten sein kann.

Ein besonderer Vorteil organischer fotovoltaischer Elemente, insbesondere organischer Solarzellen und/oder organischer Fotodioden, besteht darin, dass diese grundsätzlich flexibel ausgestaltet werden können. So kann das organische fotovoltaische Element insbesondere zumindest teilweise flexibel ausgestaltet sein, insbesondere als Folie, vorzugsweise als Kunststofffolie. So kann das organische fotovoltaische Element beispielsweise einen flexiblen Träger aufweisen, beispielsweise einen Kunststoff-Träger. Alternativ oder zusätzlich sind auch andere flexible Trägermaterialien einsetzbar, beispielsweise dünne Gläser. Auch mehrschichtige Substrat-Aufbauten sind einsetzbar, beispielsweise Kunststoff-Glas-Laminate. Auch derartige flexible Träger sind dem Fachmann aus dem Bereich der organischen Elektronik und/oder organischen Fotovoltaik grundsätzlich bekannt.

In einer besonders bevorzugten Ausgestaltung des Detektors umfasst dieser Detektor weiterhin mindestens ein Anzeigenelement, welches eingerichtet ist, um das elektrische Signal zumindest teilweise in mindestens ein für einen Benutzer wahrnehmbares Ausgangssignal, insbesondere ein optisches Ausgangssignal, umzuwandeln, insbesondere ein sichtbares optisches Ausgangssignal. Beispielsweise kann das Anzeigenelement ein oder mehrere Displays und/oder Leuchtfelder und/oder andere Arten optischer Anzeigenelemente umfassen. Alternativ oder zusätzlich zu optischen Anzeigenelementen können jedoch auch Anzeigenelemente verwendet werden, welche auf eine andere Weise für einen Benutzer wahrnehmbare Ausgangssignale erzeugen können, beispielsweise akustische Ausgangssignale und/oder haptische Ausgangssignale. Beispielsweise kann bei Wahrnehmung von ionisierender Strahlung, alternativ oder zusätzlich zu einer optischen Anzeige, einen Warnton oder eine andere Art von akustischem Warnsignal ausgegeben werden, eine Sprachausgabe erfolgen oder eine für einen Benutzer wahrnehmbare Vibration des Detektors.

Besonders bevorzugt ist es, wenn das organische fotovoltaische Element mindestens ein Array organischer fotovoltaischer Elemente umfasst, wobei auch das Anzeigenelement ein Array von Anzeigenelementen umfassen kann. In diesem Fall kann insbesondere eine Zuordnung von einem oder mehreren Feldern des Arrays der fotovoltaischen Elemente zu Feldern des Arrays der Anzeigenelemente bestehen. Dies kann beispielsweise eine 1:1-Zuordnung sein, so dass jedem Feld des Arrays der organischen fotovoltaischen Elemente jeweils genau ein Feld des Arrays der Anzeigenelemente zugeordnet wird. Auch eine Zusammenfassung mehrerer Felder des Arrays der organischen fotovoltaischen Elemente und/oder mehrer Felder des Arrays der Anzeigenelemente kann jedoch grundsätzlich erfolgen, und/oder es können einzelne oder mehrere Felder ohne Zuordnung verbleiben. Auf diese Weise kann beispielsweise jeweils das mindestens eine elektrische Signal jeweils eines Feldes oder mehrerer zusammengefasster Felder des Arrays der organischen fotovoltaischen Elemente an ein Feld oder mehrere zusammengefasste Felder des Arrays der Anzeigenelemente übergeben werden, so dass eine Zuordnung der optischen Ausgangssignale des Arrays der Anzeigenelemente zu den elektrischen Signalen des Arrays der organischen fotovoltaischen Elemente bestehen kann, die auch beispielsweise für einen Betrachter wahrnehmbar sein kann. Auf diese Weise kann beispielsweise für einen Betrachter eine Ortsauflösung visualisiert werden. Beispielsweise kann auf diese Weise eine Ortsinformation und/oder sogar eine Bildinformation einer räumlichen Verteilung der ionisierenden Strahlung generiert und an einen Benutzers des Detektors ausgegeben werden. Die Ortsinformation und/oder die Bildinformation können auch für entsprechende Bildgebungsverfahren eingesetzt werden, beispielsweise in der bildgebenden Strahlendiagnostik und/oder Röntgendiagnostik.

In Fortsetzung des oben beschriebenen Gedankens des im Wesentlichen gesamtorganischen Aufbaus des Detektors, mit Ausnahme gegebenenfalls einiger weniger Elemente, kann der Detektor vorzugsweise derart ausgestaltet sein, dass das Anzeigenelement mindestens ein organisches lichtemittierendes Element umfasst, insbesondere mindestens eine organische Leuchtdiode. Auch dieses organische lichtemittierendes Element kann grundsätzlich wieder in Array-Form angeordnet sein, beispielsweise in Form eines oder mehrerer organischer Leuchtdioden-Arrays. Insbesondere kann das organische lichtemittierende Element mindestens eine Matrix-Anzeige umfassen, beispielsweise in Form eines Passiv-Matrix-Displays und/oder eines Aktiv-Matrix-Displays. Auch das organische lichtemittierende Element kann grundsätzlich zumindest teilweise flexibel ausgestaltet sein, beispielsweise wiederum unter Verwendung eines oder mehrerer flexibler Trägermaterialien, wobei weitgehend auf die obige Beschreibung verwiesen werden kann. Somit dann der Detektor insgesamt zumindest teilweise flexibel ausgestaltet werden, wobei der Begriff "flexibel" im Rahmen der vorliegenden Erfindung grundsätzlich eine beliebige Art der Verformbarkeit in zumindest einer Dimension umfasst, vorzugsweise eine folienartige Verformbarkeit. Insbesondere kann der Detektor insgesamt eine Dicke von weniger als 3 mm aufweisen, vorzugsweise von 1 mm oder weniger oder besonders bevorzugt sogar von 500 Mikrometern oder weniger.

Unter einem organischen lichtemittierenden Element ist allgemein im Rahmen der vorliegenden Erfindung ein lichtemittierendes Element zu verstehen, also ein Element, welches Licht zumindest auch im sichtbaren Spektralbereich emittiert, welches zumindest ein organisches Material, insbesondere ein organisches Emittermaterial, aufweist. Insbesondere kann es sich dabei wiederum um ein niedermolekulares und/oder polymeres organisches Material handeln, wobei wiederum auch ein Schichtaufbau organischer Materialien, auch in Kombination gegebenenfalls mit anorganischen Materialien, eingesetzt werden kann. Wiederum ist die Verwendung organischer Materialien mit einem ausgedehnten π-Elektronensystem, beispielsweise konjugierter organischer Materialien, bevorzugt. Die organischen Materialien können dabei beispielsweise Emittermaterialien und/oder Lochtransportmaterialien und/oder Elektrontransportmaterialien umfassen sowie gegebenenfalls weitere Materialien wie beispielsweise Pufferschichten oder ähnliches. Unter organischen Leuchtdioden (OLEDs) können grundsätzlich insbesondere dünnfilmige, leuchtende Bauelemente verstanden werden, welche mindestens ein organisches Material aufweisen, insbesondere ein organisches halbleitendes Material. Die Bauelemente weisen dabei beispielsweise eine asymmetrische Kennlinie auf, insbesondere eine Diodenkennlinie. Für den Aufbau und die Funktionsweise von organischen Leuchtdioden kann wiederum auf den Stand der Technik verwiesen werden, da bekannte Aufbauprinzipien und Herstellungsverfahren zur Erzeugung organischer Leuchtdioden oder anderer Arten organischer lichtemittierender Elemente grundsätzlich auch im Rahmen der vorliegenden Erfindung eingesetzt werden können. Insbesondere lassen sich wiederum nass-chemische Herstellungsverfahren und/oder Vakuum-Herstellungsverfahren einsetzen.

Die genannten Elemente des Detektors können grundsätzlich in beliebiger Anordnung in dem Detektor umfasst sein. Voraussetzung ist lediglich, dass die von dem Szintillator erzeugte elektromagnetische Strahlung von dem organischen fotovoltaischen Element aufgenommen werden kann. Ist ein Anzeigenelement enthalten, so sollte dieses Anzeigenelement insbesondere mit dem organischen fotovoltaischen Element direkt oder indirekt, beispielsweise über eine elektronische Schaltung, verbunden sein. Grundsätzlich können die genannten Elemente insgesamt oder teilweise nebeneinander oder übereinander angeordnet sein.

Besonders bevorzugt ist es jedoch, wenn der Detektor einen Schichtaufbau aufweist, wobei der Szintillator und das organische fotovoltaische Element übereinander in Schichten angeordnet sind. Ist auch ein Anzeigenelement vorgesehen, so kann auch dieses in den Schichtaufbau integriert sein. Beispielsweise kann mindestens eine Szintillatorschicht vorgesehen sein, mindestens eine Schicht mindestens eines fotovoltaischen Elements und mindestens eine Schicht mindestens eines Anzeigenelements. Dabei ist die Reihenfolge der Schichten grundsätzlich beliebig. Insbesondere sollte jedoch, wenn ein Anzeigenelement vorgesehen ist, die mindestens eine Schicht des Anzeigenelements derart angeordnet sein, dass diese für einen Benutzer des Detektors sichtbar ist, beispielsweise als oberste Schicht des Schichtaufbaus. Vorzugsweise ist die Schicht des organischen fotovoltaischen Elements der Schicht des Szintillators benachbart angeordnet, so dass die elektromagnetische Strahlung des Szintillators von dem fotovoltaischen Element aufgenommen werden kann. Grundsätzlich können auch mehrere Elemente in einer Schicht eben angeordnet sein, so dass beispielsweise das Anzeigenelement und das organische fotovoltaische Element zumindest teilweise in einer Schicht eben angeordnet sein können. So kann beispielsweise ein Array des organischen fotovoltaischen Elements, beispielsweise ein organisches Solarzellen-Array und/oder ein organisches Fotodioden-Array, mit einem Anzeigenelement-Array in einer Schicht kombiniert werden. So können beispielsweise ein oder mehrere Felder des Array des fotovoltaischen Elements jeweils benachbart zu einem oder mehreren Feldern des Arrays des Anzeigenelements angeordnet sein. Insbesondere kann auf diese Weise ein Array eines organischen fotovoltaischen Elements mit einem Array eines organischen lichtemittierendes Elements kombiniert werden, beispielsweise ein organisches Solarzellen-Array mit einem organischen Leuchtdioden-Array.

Der Detektor in einer oder mehreren der oben beschriebenen Ausführungsformen weist gegenüber bekannten Detektoren zum qualitativen und/oder quantitativen Nachweis ionisierender Strahlung zahlreiche Vorteile auf. So kann der Grundaufbau in einer organischen Solarzelle bestehen, die in Array-Form ausgestaltet ist, in Kombination beispielsweise mit einem darüber liegenden organischen Szintillator als Block. Der Szintillator wandelt ionisierende Strahlungen in sichtbares Licht um, das beispielsweise mit den organischen Solarzellen erfasst, insbesondere gemessen werden kann. Ein organischer Transistor kann optional das gewonnene elektrische Signal verstärken. Der mindestens eine Verstärker kann weiterhin einen Analog-Digital-Wandler umfassen und/oder mit einem derartigen Analog-Digital-Wandler verbunden oder verbindbar sein. In einem derartigen Analog-Digital-Wandler können analoge elektrische Signal beispielsweise weiter verarbeitet und/oder digitalisiert werden. Durch eine Kalibrierung kann eine Dosismessung bis hin in den Prozentbereich erzielt werden.

Da der Detektor allgemein die Möglichkeit bietet, sehr dünne Materialien zu verwenden, insbesondere dünne organische Schichten, kann auch die allgemeine Absorption der ionisierenden Strahlung durch den Detektor selbst gering ausgestaltet werden. Dies lässt sich beispielsweise dadurch nutzen, dass der Detektor direkt an einem Strahlaustritt bei einem Bestrahlungssystem verwendet wird, ohne den Primärstrahl zu verändern. Dadurch lässt sich eine präzise Messung der Fluenz der ionisierenden Strahlung erzielen, beispielsweise mit einer Auflösung von weniger als 1 mm.

Auch die Verwendung flexibler Materialien, wie beispielsweise flexibler Kunststoffmaterialien, beispielsweise für das organische fotovoltaische Element und/oder das organische lichtemittierende Element bietet zahlreiche Konstruktionsvorteile. Der Detektor kann dadurch insgesamt flexibel ausgestaltet werden, beispielsweise flexibel wie ein herkömmlicher Film, und kann damit beispielsweise als Filmersatz eingesetzt werden. Damit können die Vorteile eines Films mit denen eines digitalen Detektors kombiniert werden. Herkömmliche digitale Detektoren sind in der Regel starr und dick ausgestaltet, jedoch in vielen Fällen direkt auslesbar und weisen eine geringe bis hohe Auflösung auf. Herkömmliche Filme hingegen sind flexibel und dünn, jedoch in der Regel erst nach einem Entwickeln und/oder nach einem Scannen auslesbar, weisen jedoch eine hohe Auflösung auf. Durch Verwendung eines flexiblen Detektors gemäß der vorliegenden Erfindung lassen sich die Vorteile digitaler Detektoren und herkömmlicher Filme kombinieren, so dass nunmehr, beispielsweise unter Verwendung des oben beschriebenen Anzeigenelements, ein dünner, flexibler und dennoch direkt auslesbarer Detektor mit hoher Auflösung bereitgestellt werden kann. Durch geeignete Kombination der oben beschriebenen Merkmale ist damit beispielsweise eine vollständige Ersetzung eines Filmmaterials durch den hier geschilderten Detektor, beispielsweise in Form eines elektronischen Films, möglich, so dass beispielsweise eine vollständige oder zumindest teilweise Digitalisierung üblicher Prozesse realisiert werden kann.

Ein weiterer Vorteil des vorgeschlagenen Detektors ist die optionale Kombination organischer fotovoltaischer Elemente, wie beispielsweise organischer Solarzellen, mit organischen lichtemittierenden Elementen, beispielsweise OLEDs. Beispielsweise kann das organische lichtemittierende Element, wie oben beschrieben, in einer obersten Schicht eines Schichtaufbaus eingesetzt werden, beispielsweise in Form eines Matrix-Displays. Das organische fotovoltaische Element kann beispielsweise als unterste Schicht des Schichtaufbaus eingesetzt werden. Auch andere Schichtaufbauten sind jedoch grundsätzlich möglich, wie oben dargestellt wurde. Das organische fotovoltaische Element kann dazu eingesetzt werden, um Energie an das Anzeigenelement bereitzustellen, beispielsweise an die OLED. Die Energie kann an die OLED abgegeben werden, welche daraufhin leuchtet, so dass eine direkte Auslesbarkeit gewährleistet sein kann.

Neben den vorgeschlagenen Detektoren in einer oder mehreren der beschriebenen Ausführungsformen wird weiterhin ausgenutzt, dass der Detektor grundsätzlich in beliebiger Form ausgestaltet werden kann. Beispielsweise können mit dem Detektor in einer oder mehreren der beschriebenen Formen Oberflächen beschichtet werden, die mit radioaktiven Materialien in Berührung kommen können. Befinden sich solche Materialen auf der Oberfläche, so kann der Detektor mindestens ein elektrisches Signal erzeugen, welches direkt oder indirekt an einen Benutzer ausgegeben werden kann, beispielsweise über das mindestens eine Anzeigenelement. So kann beispielsweise ein Strom in den organischen Solarzellen erzeugt werden, welcher entweder verstärkt oder direkt als Energie zum Betreiben von organischen Leuchtdioden eingesetzt werden kann.

Dementsprechend wird eine Beschichtung zum Aufbringen auf potenziellen mit ionisierende Strahlung erzeugenden Verunreinigungen kontaminierten Oberflächen vorgeschlagen, welche mindestens einen Detektor in einer oder mehreren der oben beschriebenen Ausführungsvarianten aufweist. Der Vorteil einer derartigen Beschichtung hinsichtlich der Strahlenhygiene kann darin bestehen, dass Oberflächen, welche verunreinigt sind, beispielsweise sofort aufleuchten. Das Aufleuchten kann beispielsweise erst eingestellt werden, wenn die Verunreinigungen entfernt sind. Damit kann eine visuelle Kontrolle von ansonsten unsichtbaren, ionisierende Strahlung erzeugenden Verunreinigungen realisiert werden. Dies erhöht die Sicherheit im Umgang mit entsprechenden Strahlen erheblich.

Grundsätzlich kann der Detektor, alternativ oder zusätzlich zu einer Beschichtung, jedoch allgemein auf beliebige Weise in Gegenstände integriert werden, da der Detektor hinsichtlich seiner Form- und Materialeigenschaften sehr vielseitig, insbesondere flexibel, ausgestaltet werden kann. Beispielsweise kann ein Detektor in einer oder mehreren der beschriebenen Arten in ein Gewebe integriert werden, beispielsweise in einen Strahlenschutzanzug. Weiterhin kann der Detektor beispielsweise in eine Bleischürze integriert werden. Weiterhin kann der Detektor auch beispielsweise in einen Patienten-Abdecktuch integriert werden, welches beispielsweise bei medizinischen Eingriffen verwendet werden kann. Auch auf diese Weise kann eine unmittelbare Visualisierung einer Belastung mit ionisierender Strahlung erfolgen. Weiterhin kann, alternativ oder zusätzlich, auch eine Reinigungsvorrichtung realisiert werden, welche beispielsweise als Reinigungstuch, beispielsweise als Wischtuch, ausgestaltet sein kann. Auch eine andere Ausgestaltung in Tuch-Form oder beispielsweise in Schwamm-Form ist grundsätzlich möglich. Die Reinigungsvorrichtung kann zur Reinigung von potenziellen mit ionisierende Strahlung erzeugenden Verunreinigungen kontaminierten Oberflächen eingesetzt werden. Die Reinigungsvorrichtung kann zumindest teilweise flexibel ausgestaltet sein und mindestens einen Detektor in einer oder mehreren der oben beschriebenen Ausführungsformen umfassen. Auf diese Weise können beispielsweise Tücher produziert werden, mittels derer eine unabsichtliche Strahlenexposition und/oder Kontamination mit radioaktiven Materialien schnell und zuverlässig detektiert werden kann. Beispielsweise kann die Reinigungsvorrichtung, insbesondere das Reinigungstuch, bei Detektion entsprechender Kontamination aufleuchten.

Weiterhin wird, wie oben beschrieben, ein Bestrahlungssystem vorgeschlagen. Dieses Bestrahlungssystem umfasst mindestens eine Strahlenquelle zur Erzeugung ionisierender Strahlung. Bei dieser Strahlenquelle kann es sich beispielsweise um eine radioaktive Strahlenquelle handeln, beispielsweise eine Strahlenquelle zur Erzeugung von α- und/oder β- und/oder γ- und/oder Röntgen- und/oder Neutronen-Strahlung. Alternativ oder zusätzlich kann auch eine Röntgenquelle eingesetzt werden. Weiterhin umfasst das Bestrahlungssystem mindestens einen in einem Strahlengang des Bestrahlungssystems angeordneten Detektor in einer oder mehreren der oben beschriebenen Ausführungsvarianten. Das Bestrahlungssystem kann insbesondere eingesetzt werden, um eine Emission der ionisierenden Strahlung genau zu überwachen und/oder zu steuern bzw. zu regeln. Auch eine räumliche Verteilung der Emission der ionisierenden Strahlung und/oder eine On-line-Überwachung einer Strahlendosis, einer Strahlungsintensität, einer Strahlungsverteilung oder ähnlicher Größen kann auf diese Weise realisiert werden.

Weiterhin wird ein Verfahren zur Überwachung eines Bestrahlungssystems vorgeschlagen, welches mindestens eine Strahlenquelle zur Erzeugung ionisierender Strahlung aufweist. Beispielsweise kann es sich dabei um ein Bestrahlungssystem gemäß der obigen Beschreibung handeln. Dabei wird mindestens ein Detektor in einer oder mehreren der oben beschriebenen Ausführungsvarianten verwendet und in einem Strahlengang des Bestrahlungssystems angeordnet. Insbesondere kann die Anordnung an einem Strahlaustritt der ionisierenden Strahlung erfolgen. Dies kann beispielsweise zur Messung einer Fluenz am Strahlaustritt und/oder für eine Online-Verifikation bei Bestrahlungen verwendet werden.

Weiterhin wird ein Verfahren zur Detektion von ionisierender Strahlung erzeugenden Verunreinigungen vorgeschlagen, insbesondere radioaktiven Verunreinigungen. Bei dem Verfahren wird mindestens ein Detektor in einer oder mehreren der oben beschriebenen Ausführungsformen verwendet. Der Detektor wird dabei an einen potenziellen mit den Verunreinigungen kontaminierten Gegenstand angenähert. Diese Annäherung kann bis auf einen Abstand erfolgen und/oder bis hin zu einer Berührung des Gegenstands durch den Detektor. Beispielsweise kann die Berührung in Form eines Wischens erfolgen. Der Detektor kann auch auf einen Gegenstand aufgebracht werden und/oder zum Abreiben des Gegenstands verwendet werden. Dabei wird das mindestens eine Ausgangssignals des Detektors überwacht und aus dem Ausgangssignal auf eine Anwesenheit oder Abwesenheit der Verunreinigungen geschlossen.

Die vorgeschlagenen Vorrichtungen und Verfahren bieten, über die oben bereits genannten Vorteile hinaus, die Möglichkeit, dünne, flexible Detektoren als Filmersatz in den unterschiedlichsten Anwendungen bereitstellen zu können. Insbesondere lassen sich aktive Oberflächen zur Detektion von Strahlenexpositionen realisieren. Damit lassen sich auch Anwendungen erschließen, welche bislang einer Strahlenhygiene und/oder einem Strahlenschutz aus praktischen Gründen kaum zugänglich waren. Allgemein lassen sich dabei der Strahlenschutz und/oder die Strahlenhygiene ausweiten und kostengünstiger und sicherer gestalten. Die Nutzung von flexiblen organischen Strukturen anstelle von aufwändig zu realisierenden Halbleiterdetektoren oder Ionisationskammem bietet zudem erhebliche Kostenvorteile. Zudem lassen sich Strahlenbelastungen direkt anzeigen, beispielsweise in Form einer Anzeige radioaktiver Strahlung auf Oberflächen. Radioaktive Verunreinigungen auf Oberflächen lassen sich auf diese Weise direkt visualisieren.

### Kurze Beschreibung der Figuren

Weitere Einzelheiten und Merkmale der Erfindungen ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den Figuren bezeichnen dabei gleiche oder funktionsgleiche bzw. hinsichtlich ihrer Funktionen einander entsprechender Elemente.

Im Einzelnen zeigen
- Figur 1: einen einfachen Schichtaufbau eines ersten Ausführungsbeispiels eines Detektors;
- Figur 2: eine schematische Darstellung eines möglichen organischen fotovoltaischen Ele- ments in Array-Form;
- Figur 3: ein Ausführungsbeispiel eines Detektors mit einem Schichtaufbau mit einem Ar- ray eines organischen fotovoltaischen Elements und einem Array eines organi- schen lichtemittierenden Elements;
- Figur 4: ein Ausführungsbeispiel eines Bestrahlungssystems;
- Figur 5: ein Ausführungsbeispiel eines mit einer erfindungsgemäßen Beschichtung be- schichteten Gegenstands;
- Figur 6: ein Ausführungsbeispiel einer Reinigungsvorrichtung; und
- Figur 7: ein Ausführungsbeispiel eines Detektors mit einem Schichtaufbau mit einer Isola- tionsschicht zwischen Anzeigenelement und Szintillator.

### Ausführungsbeispiele

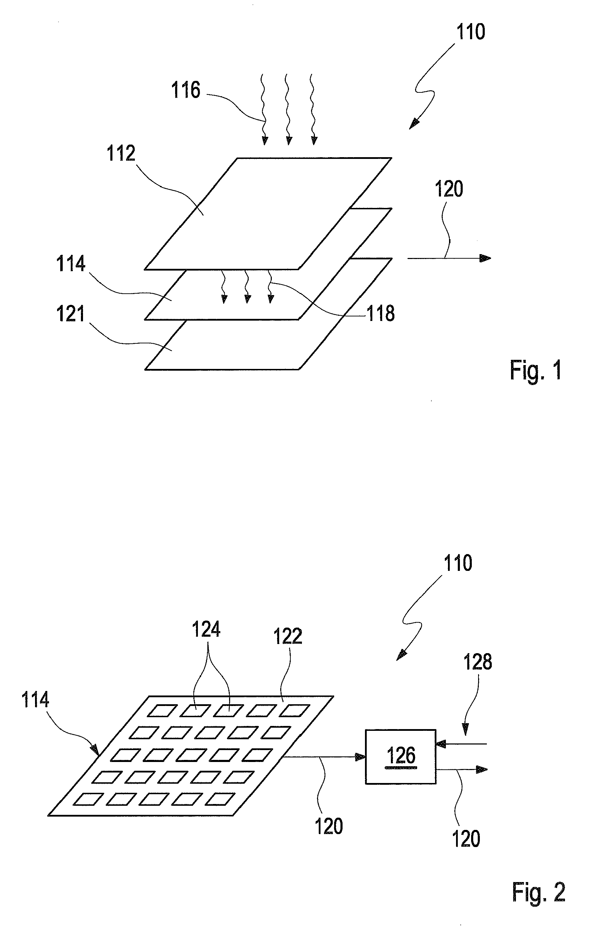

In Figur 1 ist, stark schematisiert, ein einfaches Ausführungsbeispiel eines erfindungsgemäßen Detektors 110 zum Nachweis ionisierender Strahlung dargestellt. Der Detektor 110 ist in diesem Ausführungsbeispiel als Schichtaufbau realisiert und umfasst eine Schicht eines Szintillators 112, beispielsweise eines organischen Szintillators 112, und eine Schicht eines organischen fotovoltaischen Elements 114, beispielsweise eine Schicht einer organischen Solarzelle. Der Szintillator 112 ist eingerichtet, um ionisierende Strahlung 116 zumindest teilweise zu absorbieren und in elektromagnetische Strahlung 118 umzuwandeln. Das organische fotovoltaische Element 114 ist seinerseits eingerichtet, um die organische elektromagnetische Strahlung 118 zumindest teilweise zu absorbieren und mindestens ein elektrisches Signal zu erzeugen, welches in Figur 1 symbolisch mit der Bezugsziffer 120 bezeichnet ist. Beispielsweise als unterste Schicht kann in diesem Ausführungsbeispiel des Detektors 110 optional weiterhin eine Trägerschicht vorgesehen sein, welche in Figur 1 symbolisch mit der Bezugsziffer 121 bezeichnet ist.

In Figur 2 ist eine mögliche Ausgestaltung des organischen fotovoltaischen Elements 114 gezeigt. Dieses kann beispielsweise einen flexiblen Träger 122 aufweisen, auf welchen ein Array 124 einzelner fotovoltaischer Elemente 114 aufgebracht ist. Beispielsweise kann es sich dabei um ein organisches Solarzellen-Array handeln. Dieses kann beispielsweise je nach Anwendung in geeignete Segmente segmentiert sein. Beispielsweise bietet sich für Messungen eine Arrayform mit ausfüllenden Quadraten oder eine andere Füllstrategie mit Sechsecken an. Grundlegend sollte die Überdeckung möglicht vollständig sein, um keine Messdaten zu verlieren.

Das mindestens eine fotovoltaische Element 114 und/oder das Array 124 der fotovoltaischen Elemente 114 kann beispielsweise auf der Verwendung von Fotodioden und/oder Fotoelementen basieren. Die Nutzung einer Fotodiode kann insbesondere dadurch erfolgen, dass sich bei Lichteinstrahlung ein Widerstand der Fotodiode ändert. Dies kann ausgenutzt und/oder gemessen werden, indem beispielsweise ein Strom durch die Fotodiode gemessen wird. Ein typischer Schaltungsaufbau kann beispielsweise vorsehen, dass eine Spannung an die Fotodiode angelegt wird und ein Strom zuerst durch einen Strom-Spannungs-Wandler konvertiert, die Spannung dann verstärkt und digitalisiert wird. Alternativ oder zusätzlich kann auch ein Fotoelement genutzt werden, beispielsweise eine organische Solarzelle. Hierbei können Ladungen erzeugt werden, welche dann beispielsweise in einem Kondensator zwischengespeichert werden können, analog zu einer elektrischen Schaltung eines CCD-Elements (CCD: charge-coupled device, ladungsgekoppeltes Bauteil).

Jedes einzelne fotovoltaische Element 114 kann einen Schichtaufbau aufweisen. Beispielsweise kann ein derartiger Schichtaufbau eine Kombination mindestens einer Schicht eines Elektronendonators und mindestens eine Schicht eines Elektronenakzeptors vorsehen und/oder gemischte Schichten mit beiden Eigenschaften. Beispielsweise kann ein Schichtaufbau mit mindestens einer Schicht Poly(3-hexylthiophen) (P3HT) als Elektronendonator und mindestens einer Schicht [6,6]-Phenyl-C61-butansäuremethylester (PCBM) als Elektronenakzeptor verwendet werden, beispielsweise in Form eines organischen 2-SchichtAufbaus. Dieser organische Schichtaufbau kann zwischen mindestens zwei Elektroden eingebettet werden, von denen beispielsweise mindestens eine auf einen Träger aufgebracht ist, beispielsweise einen transparenten Träger. Zwischen den beiden Elektroden (Anode, Kathode) kann eine Spannung angelegt werden. Wird ein Elektronen-Loch-Paar erzeugt, werden diese jeweils auf die entsprechende Elektrode gezogen und erzeugen einen Strom. Auch ein Aufbau ohne externe Spannung ist denkbar. Beispielsweise kann ein typischer Schichtaufbau folgende Schichtfolge vorsehen: eine Elektrode, beispielsweise eine wenige Nanometer Dicke Doppelschicht Ca/Ag mit optischer Transparenz, gefolgt von einer aktiven organischen Schicht oder Schichtfolge, gefolgt von einer optionalen Zwischenschicht zur Minimierung eines Dunkelstroms, beispielsweise Polystyrenesulphonat, beispielsweise mit einer Dicke von einem Mikrometer, gefolgt von einer zweiten Elektrode, gefolgt wiederum von einem Trägermaterial. Weiterhin sollte das System verkapselt werden, um oxidative Probleme zu vermeiden.

Der Szintillator 112 des Detektors 110 ist dabei in Figur 2 nicht dargestellt. Grundsätzlich ist dabei ein klassischer Schichtaufbau mit oben liegendem Szintillator 112 und einem darunter fotovoltaischen Element 114, beispielsweise in Form mindestens einer Photodiode und/oder mindestens einer Solarzelle, bevorzugt. Beispielsweise kann der Szintillator 112 in Form eines Blocks über das organische fotovoltaische Element 114 aufgebracht werden, beispielsweise analog zu Figur 1. Der Szintillator 112 kann aus der ionisierenden Strahlung 116 elektromagnetische Strahlung 118 erzeugen, beispielsweise sichtbares Licht, welche im fotovoltaischen Element 114 in einen Strom und/oder eine Spannung umgewandelt werden kann. Der Szintillator 112 kann in den Ausführungsbeispielen in den Figuren eins und zwei beispielsweise Anthrazen, Stilben, Terpenyl, Diphenylazethylen, Polyvinlytoluol, Toluol oder Xylol oder Kombinationen der genannten und/oder anderer Szintillatormaterialien umfassen. Weitere Szintillatormaterialien, die auch im Rahmen der vorliegenden Erfindung einsetzbar sind, sind beispielsweise in Hanno Krieger: Grundlagen der Strahlungsphysik und des Strahlenschutzes, Band 2, S. 177-178, Tabelle 2.3 offenbart.

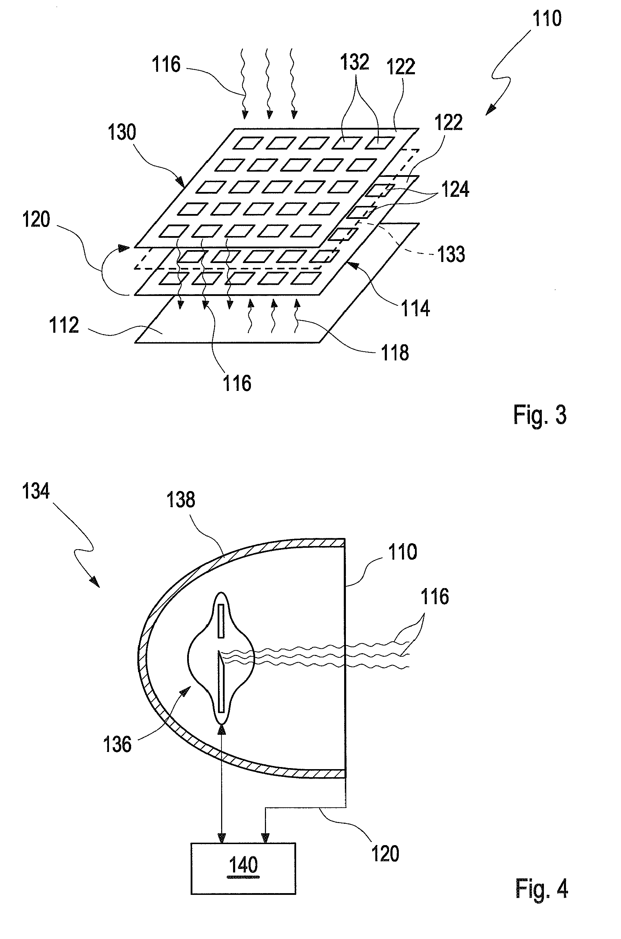

Weiterhin kann der Detektor 110 in dem in Figur 2 gezeigten Ausführungsbeispiel, wie auch in den anderen Ausführungsbeispielen gemäß den Figuren 1 und 3, ein oder mehrere weitere, in den Figuren nicht gezeigte Schichten umfassen, wie beispielsweise eine oder mehrere Trägerschichten 121 bzw. Schichten eines Trägermaterials, welche beispielsweise als unterste Schicht des Schichtaufbaus in den Figuren 2 und 3 verwendet werden können und/oder als Zwischenschichten. Die mindestens eine optionale Schicht des Trägermaterials kann weiterhin mit einem oder mehreren anderen Elementen des Detektors 110 zusammengefasst sein, beispielsweise mit dem Szintillator 112 und/oder dem fotovoltaischen Element 114 und/oder einem Array 124 der fotovoltaischen Elemente 114 und/oder mit dem unten noch näher ausgeführten Anzeigenelement 130 bzw. einem Array 132 von Anzeigenelementen 132. alternativ oder zusätzlich kann die mindestens eine optionale Schicht des Trägermaterials jedoch auch als eigenständige Schicht ausgebildet sein, beispielsweise, wie oben erläutert, als unterste Schicht eines Gesamtschichtaufbaus.

Weiterhin kann der Detektor 110 in diesem oder in anderen Ausführungsbeispielen eine Ansteuer- und/oder Auswerteelektronik 126 umfassen. Diese ist in Figur 2 symbolisch angedeutet. Beispielsweise kann diese Ansteuer- und/oder Auswerteelektronik 126 ganz oder teilweise in organischer Elektronik realisiert werden. Die Ansteuer- und/oder Auswerteelektronik 126 kann beispielsweise einen oder mehrere Verstärker und/oder einen oder mehrere Analog-Digital-Wandler und/oder ein oder mehrere Auswerteelemente umfassen, beispielsweise um das elektrische Signal 120 bereits vollständig oder teilweise innerhalb des Detektors zu verarbeiten oder vorzuverarbeiten. Auch eine Ausgabe von Dosisinformationen kann auf diese Weise erfolgen. In Figur 2 ist dabei zwischen dem elektrischen Signal 120 vor und nach der Ansteuer- und/oder Auswerteelektronik 126 nicht zu unterscheiden, wobei jedoch naturgemäß Unterschiede zwischen den elektrischen Signalen 120 bestehen können. Die Ansteuer- und/oder Auswerteelektronik 126 kann auch ganz oder teilweise in organische Elektronik realisiert werden, beispielsweise unter Verwendung eines oder mehrerer organischer Transistoren. Auch Hybridaufbauten sind grundsätzlich möglich. Die von der Ansteuer- und/oder Auswerteelektronik 126 generierten und/oder modifizierten elektrischen Signale 120 können beispielsweise über eine Schnittstelle 128 an einen Benutzer und/oder weitere Vorrichtungen ausgegeben werden. Alternativ oder zusätzlich können die elektrischen Signale 120 jedoch auch direkt oder indirekt an weitere Komponenten des Detektors 110 weitergeleitet werden, über die Schnittstelle 128 oder unter Umgehung dieser Schnittstelle 128. Beispielsweise kann, wie anhand Figur 3 näher erläutert wird, eine Übergabe der elektrischen Signale 120 an ein oder mehrere Anzeigenelemente 130 erfolgen.

In Figur 3 ist ein Ausführungsbeispiel eines erfindungsgemäßen Detektors 110 gezeigt, welcher zusätzlich mindestens ein Anzeigenelement 130 aufweist. Wiederum ist der Detektor 110 exemplarisch in einem Schichtaufbau realisiert, mit einem Szintillator 112 und einem Array 124 von fotovoltaischen Elementen, beispielsweise analog zu Figur 2. Auch das Anzeigenelement 130, welches beispielsweise als organisches lichtemittierendes Element, insbesondere als OLED, realisiert sein kann, ist in diesem Fall als Array 132 realisiert. Beispielsweise können einem oder mehreren Feldern des Arrays 124 jeweils ein oder mehrere Felder des Arrays 132 zugeordnet sein.

Die ionisierende Strahlung 116 kann beispielsweise eine oder mehrere Schichten des Schichtaufbaus durchdringen und zu dem Szintillator 112 gelangen. Der Szintillator 112 ist hier als großflächiger Szintillator dargestellt, kann jedoch, alternativ oder zusätzlich, ebenfalls ganz oder teilweise in Array-Form einzelner oder mehrerer Szintillatoren ausgestaltet sein. Alternativ oder zusätzlich kann eine dem jeweiligen Anwendungsproblem angepasste Geometrie und/oder Segmentierung des Szintillators 112 verwendet werden. Wie in Figur 3 dargestellt, wird die ionisierende Strahlung 116 in dem Szintillator 112 wiederum in elektromagnetische Strahlung 118 umgewandelt, welche wiederum in dem organischen fotovoltaischen Element 112 in elektrische Signale 120 umgewandelt werden kann. Diese elektrischen Signale 120 können, wie in Figur 3 angedeutet, direkt oder indirekt an das Anzeigenelement 130 ausgegeben werden. Dabei kann auch eine vollständige oder teilweise Zwischenverarbeitung stattfinden, beispielsweise eine Verstärkung der Signale, beispielsweise analog zu Figur 2. Auf diese Weise kann beispielsweise ein Aufleuchten des Anzeigenelements 130, beispielsweise ein großflächiges und/oder ein lokales Aufleuchten, die ionisierende Strahlung 116 und/oder ionisierende Strahlung 116 erzeugende Substanzen anzeigen. Der Detektor 110 gemäß Figur 3 kann beispielsweise als Film ausgestaltet sein, so dass dieser beispielsweise herkömmliche Strahlendetektionsfilme ersetzen kann und beispielsweise zum großflächigen oder ortsaufgelösten Nachweisen der ionisierenden Strahlen 116 eingesetzt werden kann. Durch das Aufleuchten des Anzeigenelements 130 kann die ionisierende Strahlung 116 für einen Benutzer unmittelbar visualisiert werden. Damit werden die Vorteile üblicher digitaler Detektoren mit den Vorteilen herkömmlicher Filme kombiniert.

Allgemein sei darauf hingewiesen, dass der in Figur 3 dargestellte Schichtaufbau nicht zwingend erforderlich ist. So kann der Schichtaufbau eine andere Schichtreihenfolge umfassen. Beispielsweise kann der Szintillator 112 zwischen dem organischen fotovoltaischen Element 114 und dem Anzeigenelement 130 angeordnet sein. Weiterhin kann auch jedes der dargestellten Elemente für sich wiederum einen Schichtaufbau aufweisen. So sind beispielsweise übliche organische fotovoltaische Elemente 114 und/oder übliche Anzeigenelemente 130 ihrerseits als Schichtaufbau realisiert. Beispielsweise können diese Elemente jeweils einen oder mehrere Sandwich-Aufbauten umfassen, beispielsweise mit einem Träger, zwei oder mehr Elektroden und mindestens einem dazwischen eingebetteten organischen Material. Diesbezüglich kann auf den Stand der Technik verwiesen werden.

Weiterhin kann in dem Schichtaufbau gemäß Figur 3, wie auch in anderen Schichtaufbauten, beispielsweise gemäß den Figuren 1 und 2, mindestens eine Isolationsschicht integriert werden, welche als Option in Figur 3 gestrichelt dargestellt ist und mit der Bezugsziffer 133 bezeichnet ist. Diese Isolationsschicht 133 kann beispielsweise intransparent oder mit geringer Transparenz für sichtbares Licht ausgestaltet sein und kann beispielsweise bewirken, dass sichtbares Licht von dem fotovoltaischen Element 114 ferngehalten wird oder zumindest abgeschwächt wird, bevor dieses das fotovoltaische Element 114 erreicht.

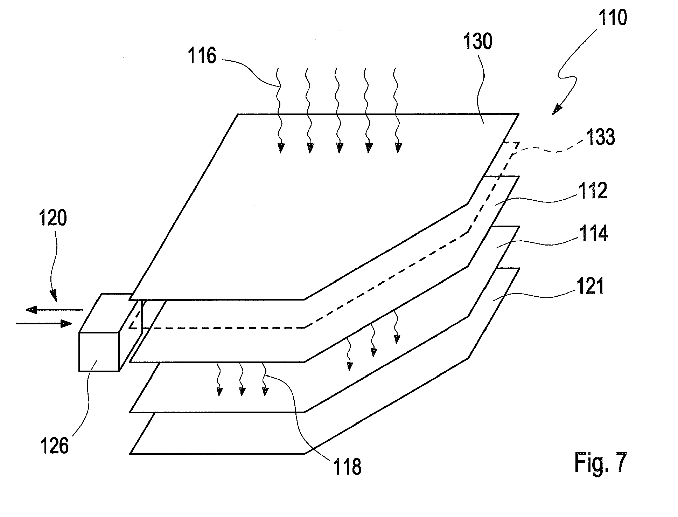

In Figur 7 ist ein weiteres Ausführungsbeispiel eines Detektors 110 dargestellt, in welchem eine Isolationsschicht 133 zum Einsatz kommen kann. In diesem Ausführungsbeispiel ist optional als unterste Schicht wiederum eine Trägerschicht 121 vorgesehen. Darüber ist eine Schicht eines organischen fotovoltaischen Elements 114 vorgesehen, beispielsweise eines großflächigen organischen fotovoltaischen Elements. Darüber ist eine Schicht des Szintillators 112 vorgesehen. Über diesem Schichtaufbau ist eine Isolationsschicht 133 vorgesehen, welche vorzugsweise zumindest teilweise transparent ist für die von oben auftreffende ionisierende Strahlung 116, so dass diese zu dem organischen fotovoltaischen Element 114 gelangen kann. Andererseits ist diese Isolationsschicht 133 jedoch zumindest teilweise intransparent für sichtbares Licht, so dass keine oder nur eine geringfügige Verfälschung des Signals des organischen fotovoltaischen Elements 114 durch das sichtbare Licht auftreten kann. Über der Isolationsschicht 133 ist dann wiederum ein Anzeigenelement 130 angeordnet, vorzugsweise in Form einer großflächigen Lichtquelle, vorzugsweise einer organischen Leuchtdiode. Diese kann durch zwei oder mehr Elektroden angesteuert sein.

Das organische fotovoltaische Element 114 in dem in Figur 7 gezeigten Schichtaufbau kann wiederum auf verschiedene Weisen ausgestaltet werden. Beispielsweise kann dieses als Photodiode und/oder als Solarzelle ausgelegt sein und/oder als ein anderes organisches fotovoltaisches Element, welches in Reaktion auf die von dem Szintillator 112 erzeugte elektromagnetische Strahlung 118 ein elektrisches Signal erzeugt. Dieses Signal kann beispielsweise in an verteilten Orten sitzenden elektronischen Schaltkreisen das Einschalten des Anzeigenelements 130, beispielsweise der OLEDs, bewirken. Das mindestens einer Anzeigenelement 130 kann dabei aufleuchten oder auch andere optische Signale emittieren, beispielsweise blinkende Signale und/oder Symbole, wie beispielsweise ein Symbol für Radioaktivität.

In Figur 4 ist schematisch ein Ausführungsbeispiel eines erfindungsgemäßen Bestrahlungssystems 134 zur Erzeugung ionisierender Strahlung 116 dargestellt. Das Bestrahlungssystem 134 umfasst eine Strahlenquelle 136, welche hier beispielsweise in einem Gehäuse 138 aufgenommen ist und welche ionisierende Strahlung 116 erzeugt. Exemplarisch ist die Strahlenquelle 136 hier als Röntgenröhre symbolisiert. Alternativ oder zusätzlich können jedoch auch andere Strahlenquellen 136 eingesetzt werden, beispielsweise α- und/oder β- und/oder γ- und/oder Röntgen- und/oder Neutronenstrahlenquellen.

Das Bestrahlungssystem 134 kann weiterhin eine Steuerung 140 umfassen, welche in Figur 4 symbolisch angedeutet ist. Im Strahlengang des Bestrahlungssystems 134 ist ein Detektor 110, beispielsweise gemäß einer oder mehreren der oben beschriebenen Ausführungsformen, angeordnet. Da der Detektor 110 beispielsweise sehr dünn ausgestaltet werden kann und beispielsweise mit lediglich geringfügig ionisierender Strahlung 116 absorbierenden Materialen hergestellt werden kann, wird durch diese Anordnung des Detektors 110 die ionisierende Strahlung 116 in ihrer Intensität und/oder räumlichen Verteilung nicht oder nur unwesentlich beeinflusst. Ein von dem Detektor 110 generiertes elektrisches Signal 120 kann beispielsweise an die Steuerung 140 und/oder eine weitere, nicht dargestellte Vorrichtung ausgegeben werden. Auf diese Weise kann beispielsweise eine Ansteuerung des Bestrahlungssystems 134 hinsichtlich einer Intensität und/oder räumlichen Verteilung der ionisierenden Strahlung 116 erfolgen. Alternativ oder zusätzlich kann mittels des Detektors 110 für einen Betrachter jedoch auch unmittelbar eine Visualisierung der ionisierenden Strahlung 116 erfolgen.

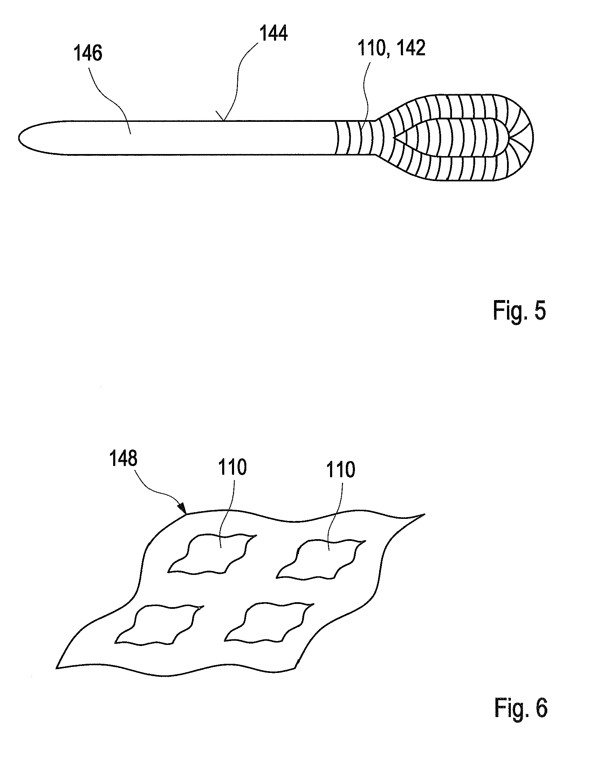

In Figur 5 ist ein Ausführungsbeispiel gezeigt, welches verdeutlicht, dass der Detektor 110 auch ganz oder teilweise in Form einer Beschichtung 142 realisiert werden kann und/oder auf andere Weise in Gebrauchsgegenstände implementiert werden kann. Die Beschichtung 142 umfasst beispielsweise einen Schichtaufbau mit einem Szintillator 112, einem organischen fotovoltaischen Element 114 und einem Anzeigenelement 130, beispielsweise jeweils in großflächiger Form. Die Beschichtung kann beispielsweise auch auf eine Oberfläche 144 eines grundsätzlich beliebigen Gegenstands 146 aufgebracht werden, welche potenziell mit ionisierende Strahlung erzeugenden Verunreinigungen kontaminiert sein kann. Im dargestellten Ausführungsbeispiel ist der Gegenstand 146 als Spatel symbolisiert. Auch andere Gegenstände 146 können jedoch eingesetzt werden, beispielsweise Gegenstände aus dem Bereich der Labordiagnostik, der Handhabung radioaktiver Substanzen, der Strahlendiagnostik oder der Strahlentherapeutik. Auf diese Weise kann, beispielsweise durch ein Aufleuchten der Oberfläche 144, eine Kontamination visualisiert werden.

In Figur 6 ist schließlich ein Ausführungsbeispiel einer Reinigungsvorrichtung 148 dargestellt. Diese Reinigungsvorrichtung 148 ist in diesem Beispiel als Tuch realisiert, mit welchem beispielsweise potenziell kontaminierten Oberflächen gewischt werden können. In diese Reinigungsvorrichtung 148 sind ein oder mehrere Detektoren 110 integriert, wobei die Reinigungsvorrichtung 148 auch vollständig als Detektor 110 ausgestaltet sein kann. Hier kann insbesondere wiederum die Option einer flexiblen Ausgestaltung der Detektoren 110 ausgenutzt werden. Grundsätzlich können die Detektoren 110 wiederum gemäß der obigen Beschreibung ausgestaltet werden. Durch Wischen potenziell kontaminierter Oberflächen mit der Reinigungsvorrichtung 148 können dabei Kontaminationen mit ionisierender Strahlung erzeugenden Verunreinigungen beispielsweise wiederum unmittelbar für einen Benutzer visualisiert werden, beispielsweise indem die Reinigungsvorrichtung 148 lokal oder großflächig aufleuchtet.

### Bezugszeichenliste

- 110: Detektor zum Nachweis ionisierender Strahlung
- 112: Szintillator
- 114: Organisches fotovoltaisches Element
- 116: Ionisierende Strahlung
- 118: Elektromagnetische Strahlung
- 120: Elektrisches Signal
- 121: Trägerschicht
- 122: Flexibler Träger
- 124: Array fotovoltaischer Elemente
- 126: Ansteuer- und/oder Auswerteelektronik
- 128: Schnittstelle
- 130: Anzeigenelement
- 132: Array von Anzeigenelementen
- 133: Isolationsschicht
- 134: Bestrahlungssystem
- 136: Strahlenquelle
- 138: Gehäuse
- 140: Steuerung
- 142: Beschichtung
- 144: Oberfläche
- 146: Gegenstand
- 148: Reinigungsvorrichtung

## Patentansprüche

1. Detektor (110) zum Nachweis ionisierender Strahlung (116), umfassend mindestens einen Szintillator (112), wobei der Szintillator (112) eingerichtet ist, um die ionisierende Strahlung (116) in elektromagnetische Strahlung (118) umzuwandeln, insbesondere in sichtbares, ultraviolettes oder infrarotes Licht, wobei der Detektor (110) weiterhin mindestens ein organisches fotovoltaisches Element (114) umfasst, wobei das organische fotovoltaische Element (114) eingerichtet ist, um die elektromagnetische Strahlung (118) in mindestens ein elektrisches Signal (120) umzuwandeln.

2. Detektor (110) nach dem vorhergehenden Anspruch, wobei der Szintillator (112) mindestens ein organisches Szintillatormaterial umfasst.

3. Detektor (110) nach einem der vorhergehenden Ansprüche, wobei das organische fotovoltaische Element (114) mindestens eine organische Solarzelle und/oder mindestens eine organische Fotodiode, insbesondere mindestens ein organisches Solarzellen-Array und/oder mindestens ein organisches Fotodioden-Array, umfasst.

4. Detektor (110) nach einem der vorhergehenden Ansprüche, weiterhin umfassend mindestens einen Verstärker zur Verstärkung des elektrischen Signals (120), insbesondere einen mindestens ein organisches elektronisches Element und vorzugsweise einen organischen Transistor, umfassenden Verstärker.

5. Detektor (110) nach einem der vorhergehenden Ansprüche, wobei der Detektor (110) eingerichtet ist, um aus dem elektrischen Signal (120) eine Dosisinformation über eine Strahlungsdosis der ionisierenden Strahlung (116) zu erzeugen.

6. Detektor (110) nach einem der vorhergehenden Ansprüche, wobei das organische fotovoltaische Element (114) zumindest teilweise flexibel ausgestaltet ist, insbesondere als Folie, vorzugsweise als Kunststofffolie.

7. Detektor (110) nach einem der vorhergehenden Ansprüche, weiterhin umfassend mindestens ein Anzeigenelement (130), wobei das Anzeigenelement (130) eingerichtet ist, um das elektrische Signal (120) in mindestens ein für einen Benutzer wahrnehmbares Ausgangssignal, insbesondere ein optisches Ausgangssignal, umzuwandeln.

8. Detektor (110) nach dem vorhergehenden Anspruch, wobei das organische fotovoltaische Element (114) mindestens ein Array (124) organischer fotovoltaischer Elemente umfasst, wobei das Anzeigenelement (130) ein Array (132) von Anzeigenelementen umfasst, wobei eine Zuordnung von Feldern des Arrays (124) der organischen fotovoltaischen Elemente zu Feldern des Arrays (132) der Anzeigenelemente besteht.

9. Detektor (110) nach einem der beiden vorhergehenden Ansprüche, wobei das Anzeigenelement (130) mindestens ein organisches lichtemittierendes Element umfasst, insbesondere mindestens eine organische Leuchtdiode, insbesondere ein organisches Leuchtdioden-Array.

10. Detektor (110) nach einem der vorhergehenden Ansprüche, wobei der Detektor (110) einen Schichtaufbau aufweist, wobei der Szintillator (112) und das organische fotovoltaische Element (114) und vorzugsweise das Anzeigenelement (130) in Schichten übereinander angeordnet sind.

11. Beschichtung (142) zum Aufbringen auf potentiell mit ionisierender Strahlung (116) erzeugenden Verunreinigungen kontaminierten Oberflächen (144), wobei die Beschichtung (142) mindestens einen Detektor (110) nach einem der vorhergehenden, einen Detektor (110) betreffenden Ansprüche aufweist.

12. Reinigungsvorrichtung (148), insbesondere Reinigungstuch, zur Reinigung potentiell mit ionisierende Strahlung (116) erzeugenden Verunreinigungen kontaminierten Oberflächen, wobei die Reinigungsvorrichtung (148) zumindest teilweise flexibel ausgestaltet ist und mindestens einen Detektor (110) nach einem der vorhergehenden, einen Detektor (110) betreffenden Ansprüche aufweist.

13. Bestrahlungssystem (134), umfassend mindestens eine Strahlenquelle (136) zur Erzeugung ionisierender Strahlung (116), weiterhin umfassend mindestens einen in einem Strahlengang des Bestrahlungssystems (134) angeordneten Detektor (110) nach einem der vorhergehenden, einen Detektor (110) betreffenden Ansprüche.

14. Verfahren zur Detektion von ionisierende Strahlung (116) erzeugenden Verunreinigungen, insbesondere radioaktiven Verunreinigungen, wobei mindestens ein Detektor (110) nach einem der vorhergehenden, einen Detektor (110) betreffenden Ansprüche an einen potentiell mit den Verunreinigungen kontaminierten Gegenstand angenähert wird, wobei insbesondere der Detektor (110) auf den Gegenstand aufgebracht wird und/oder zum Abreiben des Gegenstands verwendet wird, wobei mindestens ein Ausgangssignal des Detektors (110) überwacht wird und aus dem Ausgangssignal auf eine Anwesenheit oder Abwesenheit der Verunreinigungen geschlossen wird.

15. Verfahren zur Überwachung eines Bestrahlungssystems (134), wobei das Bestrahlungssystem (134) mindestens eine Strahlenquelle (136) zur Erzeugung ionisierender Strahlung (116) aufweist, wobei mindestens ein Detektor (110) nach einem der vorhergehenden, einen Detektor (110) betreffenden Ansprüche in einem Strahlengang des Bestrahlungssystems (134) angeordnet wird, insbesondere an einem Strahlaustritt der ionisierenden Strahlung (116).
